# EUROPEAN PATENT APPLICATION

(11) **EP 3 586 877 A1**
(43) Date of publication of application: **01.01.2020**
(21) Application number: 18777423.7
(22) Date of filing: 29.03.2018
(51) Int. Cl.: A61K 48/00, A61K 38/51, A61K 35/74, A61P 35/00, A61P 35/04

(54) **APPLICATIONS OF GENETICALLY ENGINEERED BACTERIA VNP20009-M IN PREPARATION OF DRUGS FOR PREVENTING AND TREATING LUNG CANCER**

(30) Priority: 01.04.2017 CN 201710213446
(71) Applicant: Guangzhou Sinogen Pharmaceutical Co., Ltd, Guangzhou, Guangdong 510006 (CN)
(72) Inventor: ZHAO, Allan Zijian, Guangdong 510015 (CN); LIN, Yan, Guangdong 510015 (CN); ZHOU, Sujin, Guangdong 510015 (CN); LI, Fanghong, Guangdong 510015 (CN)
(74) Representative: Lavoix
(86) International application number: PCT/CN2018/081115
(87) International publication number: WO 2018/177374

(57) **Abstract**

Provided are applications of genetically engineered bacteria VNP20009-M in the preparation of drugs for preventing and treating lung cancer.

## Description

### TECHNICAL FIELD

The present invention belongs to the technical field of genetic engineering drugs and particularly relates to new application of genetically engineered bacteria VNP20009-M in preparation of drugs for preventing and treating lung cancer.

### BACKGROUND

Cancers have become an important cause of human death with cancer incidence rates increasing by 33% from 2005 to 2015. The World Cancer Report 2014 published by the World Health Organization (WHO) predicts a rapid increase in world cancer cases, from 14 million in 2012 to 19 million in 2025 year by year and reaching 24 million by 2035.

Among the cancers, lung cancer is one of the most malignant tumors, threatening people's health and life. The incidence rate and mortality rate are the highest. In 2012 alone, almost 1.6 million people worldwide died of lung cancer. The 2015 Annual Report of the Cancer Registration Center of China shows that the incidence rate and the mortality rate of lung cancer are also the highest in China. There are more than 700,000 new cases of lung cancer in China and the death toll is more than 600,000. Most patients with lung cancer are discovered late and only adapted for drug treatment. Traditional treatments for lung cancer include local treatments (including surgery, radiotherapy, etc.) and systemic treatments (including traditional chemotherapy, molecular targeted drug therapy, etc.). Although modern medical researches have made great progress, many targeted drugs such as gefitinib, erlotinib, etc. have been invented, and lung cancer has made some progress in the radiotherapy, chemotherapy and surgery, the overall prognosis is still poor. According to statistics in 2015, the 5-year survival rate of patients with lung cancer in China is only 16.1%. Therefore, finding new ways to treat lung cancer has become a problem that scientists need to solve urgently.

The prior art shows that methionine dependence is a characteristic of most tumor cells, which is manifested by excessive demands for methionine by the tumor cells and cell proliferation is inhibited when culture is conducted in a methionine removed or precursor homocysteine substituted culture medium; while in the presence of the methionine, the cells can grow normally, including more than ten malignant tumor cells of prostate cancer, breast cancer, lung cancer, etc. However, there is no methionine dependence in normal cells. The method that causes methionine deficiency mainly includes removing the methionine from diet or decomposing the methionine by using methioninase. However, limiting intake of the methionine in diet alone has a limited effect on lowering the methionine level, and long-term limiting on the methionine intake can cause body malnutrition and metabolic disorders. Compared to the diet-limited methionine intake, the use of the methioninase does not cause excessive metabolic problems and has an anti-tumor effect.

Salmonella is a group of Gram-negative and invasive intracellular facultative anaerobic bacteria parasitized in intestines of humans and animals. Among the salmonella, a known bacterium strain VNP20009 is a vector with high tumor targeting properties, safety, and antitumor effects. The VNP20009 has significant tumor growth inhibition effects on various mouse solid tumor models of malignant melanoma, lung cancer, etc. Two phase I clinical studies conducted in the United States show that the VNP20009 can be used in the human body, has safety, but shows no antitumor effects.

### SUMMARY

To this end, a technical problem to be solved by the present invention is to provide new application of genetically engineered bacteria VNP20009-M in preparation of drugs for preventing and treating lung cancer.

In order to solve the above technical problem, the present invention discloses the application of the genetically engineered bacteria VNP20009-M in the preparation of the drugs for preventing and treating the lung cancer.

Further, the lung cancer includes lung primary tumor, recurrent tumor after lung cancer surgery and lung cancer metastatic tumor.

Further, the lung cancer includes non-small cell lung cancer, small cell lung cancer, etc.

The non-small cell lung cancer originates from epithelial cells and is a major type of the lung cancer, including squamous carcinoma, adenocarcinoma, adenosquamous carcinoma, large cell lung cancer or undifferentiated carcinoma.

The squamous carcinoma is the most common type of the lung cancer, accounting for about 50%. A majority of the patients are more than 50 years old and males are the majority. Most of the squamous carcinoma originate from larger bronchus, are often central type lung cancer, and have sensitivity to radiotherapy and chemotherapy less than that of the undifferentiated carcinoma.

The adenocarcinoma originates from bronchial mucosa epithelium, a small number of the adenocarcinoma originates from mucous glands of large bronchus, and the adenocarcinoma is relatively common in females. There are no obvious clinical symptoms in an early stage. Local infiltration or hematogenous metastasis can occur when the adenocarcinoma is discovered. The adenocarcinoma is easy to metastasize to organs of the liver, the brain, the bones, etc. in clinical practice and may also involve pleura to cause pleural effusion. The adenocarcinoma is less sensitive to radiation therapy.

The adenosquamous carcinoma and large cell carcinoma have a high degree of malignancy and a low degree of differentiation, and are prone to brain metastasis, poor in therapeutic effects and poor in prognosis. At present, treatments of large cell lung cancer are mainly based on comprehensive treatments in clinical practice and the effect of simple surgery or radiotherapy and chemotherapy is poor.

The undifferentiated carcinoma has an incidence rate ranking only second to the squamous carcinoma, is common in males, has an earlier age of onset, is high in degree of malignancy, grows fast, relatively early shows wide lymphatic and hematogenous metastasis, is relatively sensitive to radiotherapy and chemotherapy, and has the worst prognosis in the various types of lung cancer.

The small cell carcinoma, also known as small cell neuroendocrine carcinoma, is the most malignant type of lung cancer. This type of the lung cancer grows fast, metastasizes early, often metastasizes to organs of the brain, the liver, the bones, the adrenal gland, etc., has a survival period of less than 1 year commonly, and is poor in a surgical resection effect and sensitive to radiotherapy and chemotherapy, often accompanied by strong toxic and side effects and complications during the radiotherapy and chemotherapy, and relatively poor in prognosis.

Preferably, the genetically engineered bacteria VNP20009-M have a minimum effective administration dose of 3.5^{∗}10⁷ CFU/M².

The genetically engineered bacteria VNP20009-M used for cancer prevention and treatment can be administered by various routes including but not limited to oral administration, local administration, injection administration (including but not limited to transvenous, peritoneal, subcutaneous, intramuscular, intratumoral administrations), etc.

The present invention also discloses application of the genetically engineered bacteria VNP20009-M in preparation of a methioninase agent. The genetically engineered bacteria VNP20009-M have relatively high methioninase activity and can be used for the preparation of the methioninase agent.

The methioninase agent can be administered by various routes including but not limited to oral administration, local administration, injection administration (including but not limited to transvenous, peritoneal, subcutaneous, intramuscular, intratumoral administrations), etc.

As known in the prior art, the genetically engineered bacteria VNP20009-M of the invention are a known bacterium strain, and properties, shapes, and construction methods of the VNP20009-M are all as described in Chinese Patent No.CN105983103A.

The genetically engineered bacteria VNP20009-M are attenuated salmonella typhimurium VNP20009 cloned with a L-methioninase gene.

Further, the genetically engineered bacteria VNP20009-M are attenuated salmonella typhimurium VNP20009 carrying a plasmid, wherein the plasmid is cloned with the L-methioninase gene.

The plasmid includes but is not limited to a pSVSPORT plasmid, a pTrc99A plasmid, a pcDNA3.1 plasmid, a pBR322 plasmid or a pET23a plasmid.

The genetically engineered bacteria VNP20009-M are constructed by subcloning the L-methioninase gene into the plasmid to obtain L-methioninase expression plasmid, and electrotransforming the L-methioninase expression plasmid into the attenuated salmonella typhimurium VNP20009.

Most preferably, in the construction process of the genetically engineered bacteria VNP20009-M, when the pSVSPORT plasmid is selected, the L-methioninase gene is subcloned into the plasmid to obtain the L-methioninase expression plasmid, and then the L-methioninase expression plasmid is electrotransformed into the attenuated salmonella typhimurium VNP20009 to obtain the genetically engineered bacteria.

Wherein, electrotransformation is conducted under a voltage of 2,400 V, a resistance of 400 Ω, a capacitance of 25 µF and a discharge time of 4 ms.

The present invention discloses the new application of the genetically engineered bacteria VNP20009-M for treating the lung cancer on the existing basis, the genetically engineered bacteria VNP20009-M can effectively kill lung cancer tumor cells, eliminate lung cancer tumor lesions, and have better killing effects and better therapeutic effects for primary lung cancer, recurrent lung cancer after surgery and lung cancer metastasized tumor cells to other sites; and besides, the genetically engineered bacteria have no obvious toxic and side effects on the human body and provide safe and effective new methods for the treatment of the lung cancer.

### BRIEF DESCRIPTION OF THE DRAWINGS

In order to make the content of the present invention easier to understand, the present invention will be further described in detail below with reference to the embodiments of the present invention accompanying with the drawings, wherein
Figure 1 is a 1% agarose gel electrophoretogram of plasmid pSVSPORT-L-methioninase enzyme cutting identification;
Figure 2 is a diagram showing results of methioninase expression identified by Western blot according to the present invention;
Figure 3 is a diagram showing results of detection of methioninase activity in salmonella according to the present invention;
Figure 4 shows a condition of new lesions in the neck of the patient in the embodiment 2;
Figure 5 shows a biopsy cell smear of the patient's mass part in the embodiment 2;
Figure 6 shows a condition of the mass of the patient after 3 weeks of treatment in the embodiment 2;
Figure 7 shows a condition of the mass of the patient after 5 weeks of treatment in the embodiment 2;
Figure 8 shows a condition of the original lesion site of the patient after 12 weeks of treatment in the embodiment 2;
Figure 9 is a cytological smear of the inside of the mass of the patient after 1 week of treatment in the embodiment 2;
Figure 10 is a cytological smear of the inside of the mass of the patient after 3 weeks of treatment in the embodiment 2.

### DETAILED DESCRIPTION

**Embodiment 1** Construction of genetically engineered bacteria VNP20009-M The construction method and processes of the genetically engineered bacteria VNP20009-M of the present invention are as described in the examples of the Chinese Patent No.CN105983103A.

### (1) Construction of plasmid expressing L-methioninase gene

The L-methioninase (GenBank: L43133.1) gene is firstly synthesized, the synthesized gene is subcloned into a pUC57 plasmid (GenScript), then the pUC57 plasmid subcloned with the L-methioninase gene is subcloned into a pSVSPORT plasmid (Invitrogen) by Kpn I and Hind III enzyme cutting sites to obtain a pSVSPORT-L-methioninase expression plasmid. The specific construction processes are as follows:
the pSVSPORT plasmid is subjected to Kpn I and Hind III double enzyme cutting. An enzyme cutting system comprises 2 µg of plasmid DNA, 3 µL of 10^{∗}buffer, 1.5 µL of Kpn I enzyme, 1.5 µL of Hind III enzyme, and ddH2O added to supplement sufficiently a volume to 30 µL, and warm bath is conducted at 37 °C for 3 h. Then the enzyme cutting system is separated by electrophoresis in 1% agarose gel, a DNA band of 4.1 kb is cut out, and the DNA is purified by a gel recovery and purification kit.

A DNA fragment of a L-methioninase coding region is obtained by whole-genome synthesis, the obtained DNA fragment is subcloned into the pUC57 plasmid (GenScript), and the pUC57 plasmid subcloned with the DNA fragment is subjected to the Kpn I and Hind III double enzyme cutting. An enzyme cutting system comprises 3 µg of plasmid DNA, 3 µL of 10^{∗}buffer, 1.5 µL of Kpn I enzyme, 1.5 µL of Hind III enzyme, and ddH2O added to supplement sufficiently a volume to 30 µL, and warm bath is conducted at 37 °C for 3 h. Then the enzyme cutting system is separated by electrophoresis in 1% agarose gel, a DNA band of 1.2 kb is cut out, and the DNA is purified by the gel recovery and purification kit.

The pSVSPORT (Kpn I/Hind III) and the DNA fragment of the L-methioninase coding region (Kpn I/Hind III) are ligated, and during the ligation reaction, 2 µL of the vector, 6 µL of the inserting fragments and 1 µL of T4 DNA ligase are added, and warm bath is conducted at 16 °C for 16 h.

A ligation product is transformed into competent cells of E. coli DH5α (Takara). A tube of 50 µL of DH5α competent cells is taken to be placed on ice, after the ice is melt, 5 µL of the above-mentioned ligation product is added into the DH5α competent cells, slight flipping and uniform mixing are conducted, and then the mixture is incubated on the ice for 30 min; heat shock is conducted at 42 °C for 60 s and then standing on the ice is conducted for 2 min; 500 µL of a non-resistant LB liquid medium is added, shaking culture is conducted at 37 °C for 1 h, then the material after shaking culture is applied on an ampicillin-containing resistant LB culture medium plate and culturing overnight is conducted.

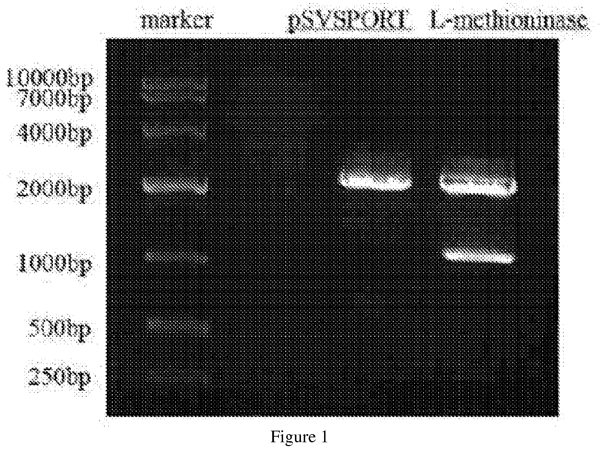

After clones are grown, monoclonal colonies are picked into 3 mL of ampicillin-containing LB culture liquid, shaking culture is conducted at 37 °C for 16 h, plasmid DNA is extracted and identified by Kpn 1 and Hind lll enzyme cutting, and in the positive clones, two DNA bands of 4.1 kb and 1.2 kb are obtained as shown in Figure 1. Then further sequencing confirms that the sequences of the positive clones are completely correct.

### (2) Constructions of VNP20009 bacteria carrying a plasmid and VNP20009 bacteria carrying a plasmid cloned with a L-methioninase gene

pSVSPORT and pSVSPORT-L-methioninase expression plasmids are respectively electrotransformed into the VNP20009 bacterium strain (YS1646, ATCC No.202165) which are respectively named as VNP20009-V and VNP20009-M. The specific construction processes are as follows:
competent bacteria VNP20009 are placed on ice, after the ice is melted, the competent bacteria VNP20009 are transferred to a pre-cooled electric rotating cup, 2 µL of the plasmid is added into the electric rotating cup, slight flipping and uniform mixing are conducted, and incubation is conducted on ice for 1 min. The electric rotating cup is put into an electric rotating instrument, and conditions are set as a voltage of 2,400 V, a resistance of 400 Ω, a capacitance of 25 µF and a discharge time of 4 ms. 1 mL of a SOC culture medium is added immediately after electric shock, and gentle and even mixing is conducted. Shaking culture is conducted at 37 °C for 1 h; and after a pipettor is used to precipitate and blow the bacteria evenly, the bacteria are applied on an ampicillin-containing resistant LB-O culture medium plate. The plate is then put in an incubator for culture at 37 °C for 16 h. After the VNP20009-V and VNP20009-M are cultured with the LB-O, the plasmid is extracted and identified by enzyme cutting to be correct.

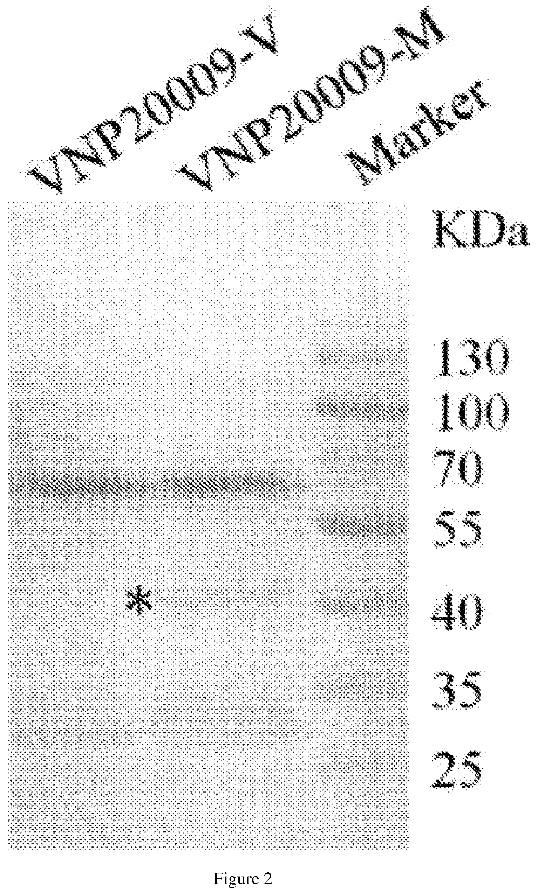

1x10⁸ of salmonella are taken, proteins are extracted by a protein lysate, 10% SDS-PAGE electrophoresis is conducted, then electrotransformation to a PVDF membrane in an ice bath under stable pressure is conducted, after BSA room temperature sealing is conducted for 1 h, TBST rinsing is conducted for 3^{∗}5 min, a rabbit anti-L-methioninase antibody is added (1:1000), and incubation is conducted overnight at 4 °C. The TBST rinsing is conducted for 3 times with 5 min each time, then a HRP-labeled anti-rabbit secondary antibody (1:10000) is added, incubation is conducted at room temperature for 1 h, the TBST rinsing is conducted for 3 times with 5 min each time, and ECL chemiluminescence developing is conducted. The results are shown in Figure 2, a specific band is observed at a molecular weight of about 43 kD, indicating that the expression level of the L-methioninase is significantly increased in the VNP20009-M compared with the VNP20009 and VNP20009-V.

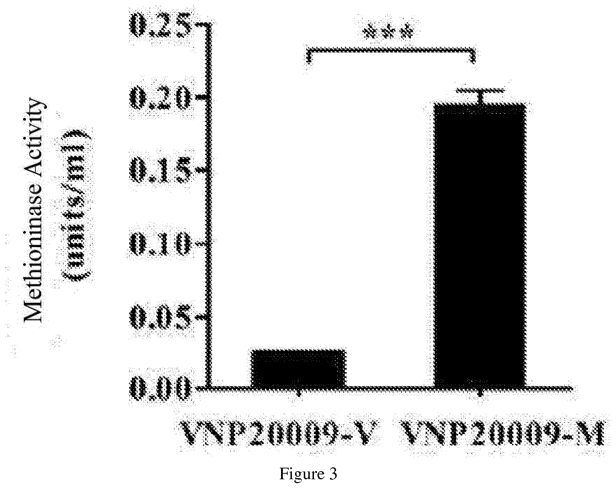

L-methionine and pyridoxal are respectively mixed with the VNP20009-V and VNP20009-M bacteria. After incubation is conducted at 37 °C for 10 min, termination is conducted with 50% trichloroacetic acid, centrifugation is conducted, a supernatant is taken, the supernatant is mixed fully and evenly with 3-methyl-2-benzothiazolinone hydrazone hydrochloride hydrate (MBTH), after incubation is conducted at 50 °C for 30 min, absorbance at 320 nm is measured, and the amount of enzyme used for catalytic conversion of 1 µmol of α-ketobutyric acid per minute is defined as 1 enzyme activity unit. Results show (as shown in Figure 3) that the activity of the methioninase in the salmonella VNP20009-M is 10 times higher than that of VNP20009-V.

Thus, the constructed genetically engineered salmonella VNP20009-M has a relatively high methioninase activity and can be used for preparation of a methioninase agent.

### Embodiment 2 Antitumor effect of genetically engineered bacteria VNP20009-M

### 1) Past medical history and diagnosis

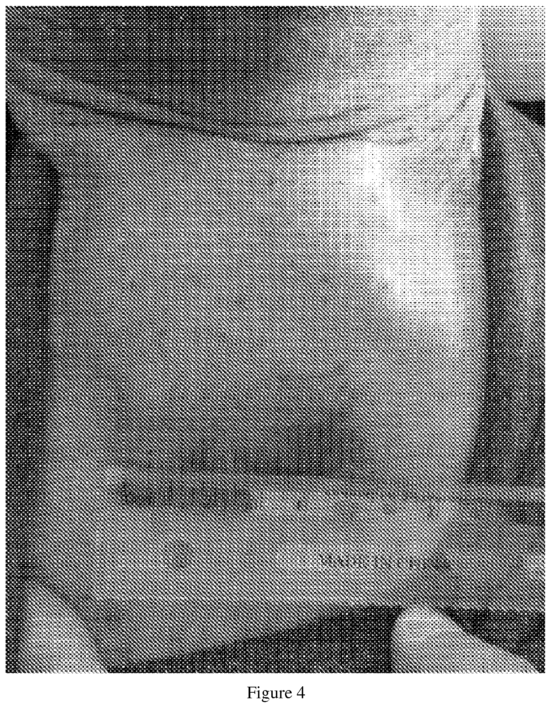

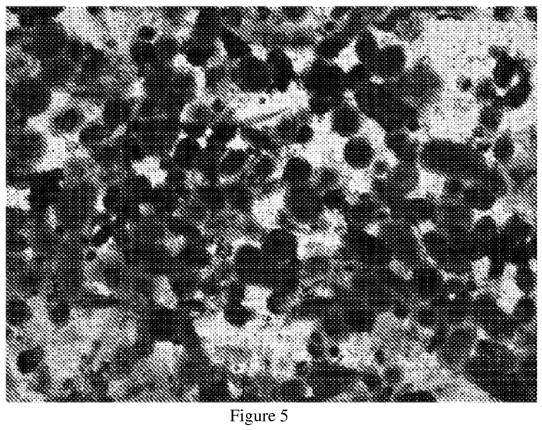

After a clinical male patient, 73 years old, undergoes a thoracoscopic right lung lower lobe squamous cell carcinoma radical operation for 5 months, a new mass is found in the neck (as shown in Figure 4). The size of the lesion mass at the neck is measured about 8 cm^{∗}9 cm. Mass part biopsy is identified as cancer cells by a cell smear (as shown in Figure 5), and besides, a bone ECT shows multiple active bone metabolism and a chest CT examination report shows sternal destruction with mass formation.

According to the patient's past treatments and related examinations, the patient is diagnosed as recurrence and metastasis after the right lung lower lobe squamous cell carcinoma operation, but there is no standard treatment plan in clinic.

### 2) Treatment plan

The diluted VNP20009-M is evenly injected into the tumor site in multi-points and during the first time, 6^{∗}10⁷ cfu (about 3.5^{∗}10⁷ cfu/m²) of the VNP20009-M is given. After a one-week interval, a second administration is continued to be conducted and a total amount of the drug is increased to 9^{∗}10⁷ cfu (about 5^{∗}10⁷ cfu/m²). An intratumoral injection is conducted as well and even and multi-point drug injection is conducted. After a one-week interval, a third administration is conducted with the same method and dose as the 2^{nd} time. After a 10-day interval, a fourth administration is conducted, a drug concentration is increased to 6^{∗}10⁷ cfu/m² and the intratumoral administration is conducted. After a 10-day interval, a fifth administration is continued to be conducted with the same method and dose as the 4^{th} time. A specific implementation plan is shown in Table 1 below.

**Table 1 Treatment implementation plan**

| Times | Time | Dose |
|---|---|---|
| 1^{st} | 0 day | 3.5^{∗}10⁷cfu/m² |
| 2^{nd} | 7^{th} day | 5^{∗}10⁷cfu/m² |
| 3^{rd} | 14^{th} day | 5^{∗}10⁷cfu/m² |
| 4^{th} | 24^{th} day | 6^{∗}10⁷cfu/m² |
| 5^{th} | 34^{th} day | 6^{∗}10⁷cfu/m² |

### 3) Efficacy

### 3.1 Changes of lesion sizes

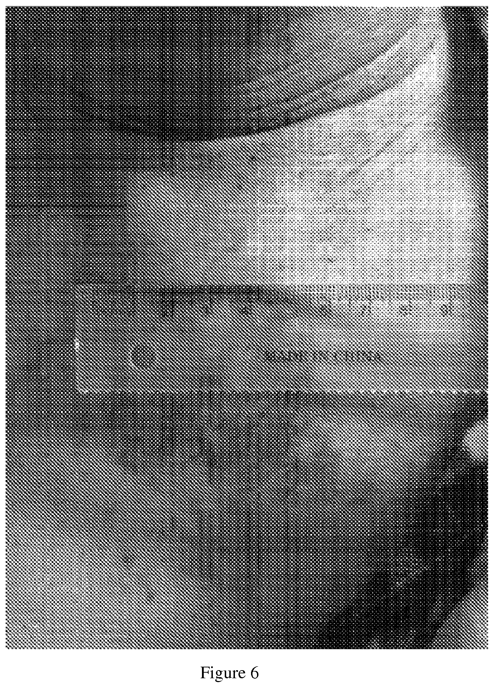

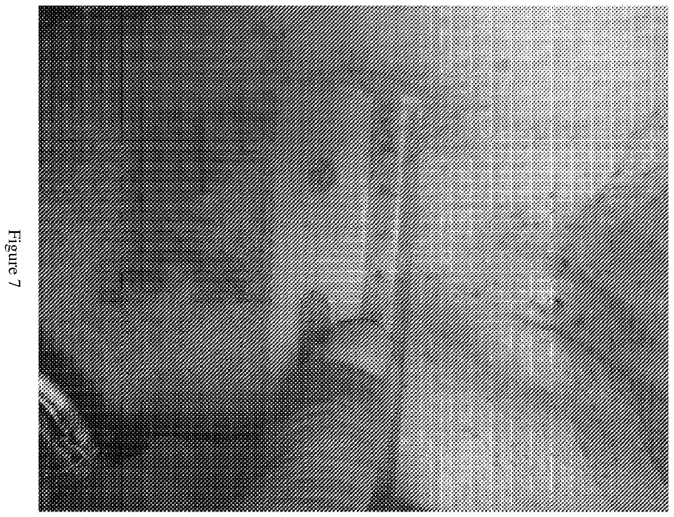

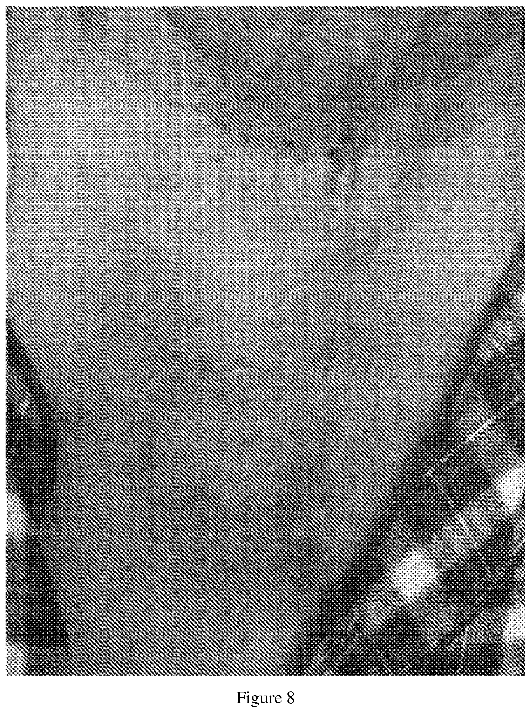

The size of the lesion mass at the neck is measured to be approximately 8 cm^{∗}9 cm before treatment (as shown in Figure 4). After 3 weeks of the treatment, the mass is significantly reduced (as shown in Figure 6). After 5 weeks of the treatment (the end of 5-times treatments), the mass basically disappears (as shown in Figure 7). After 12 weeks of the treatment, there is no abnormal change in the neck clavicle, i.e., the original lesion part (as shown in Figure 8).

### 3.2 Changes inside the mass

Before the treatment, the inside of the mass is of a cystic structure and more severely abnormal-shaped cells, i.e., tumor cells are found by a cytological smear analysis (as shown in Figure 5).

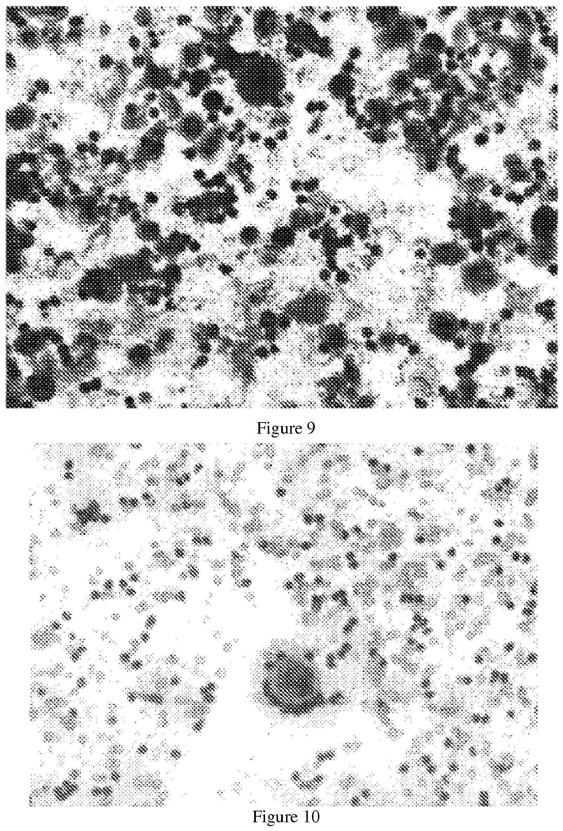

One week after the treatment (once treatment), the mass is significantly softened (as shown in Figure 9) and a large number of neutrophils and a small number of tumor cells are found by a cytological smear. 10 days after the treatment (twice treatment), about 40 mL of effusion is extracted; 2 weeks after the treatment (twice treatment), the inside of the mass is further liquefied and about 90 mL of the effusion is extracted; 3 weeks after the treatment (the treatments for the third time), the tumor is shrunk (as shown in Figure 10), about 45 mL of the effusion is continuously extracted, a large number of inflammatory cells and a very small number of tumor cells are found by a cytological smear, and it is observed that the inflammatory cells are wrapped around the periphery of the tumor cells. One month after the treatment (the treatments for the fourth time), the tumor is further reduced and the extracted effusion is reduced to about 20 mL. After the end of the 5-times treatments, no effusion is detected and the mass is eliminated.

The above results indicate that the injection of the genetically engineered bacteria VNP20009-M of the present invention into the inside of the tumor lesion can induce local inflammatory cell infiltration, thereby exerting a function of killing the tumor cells.

### 3.3 Side effects

On the day of each treatment, 9-10 hours after the injection, the patient has a fever of about 38 °C and can restore normal body temperature by physical cooling. On the day, sometimes nausea and vomiting are accompanied for about 10 minutes. Other than that, there is no other abnormal feeling. During the treatment, various indicators of liver and kidney functions are examined and the results are shown in Table 2 below. Results show that the various indicators of the liver and kidney functions of the patient are all in the normal range. The above results indicate that the VNP20009-M has no obvious toxicity to the human body.

**Table 2 Results of various examination indicators of the patient**

| Indicators | Before the treatment | 1 week after the treatment | 2 weeks after the treatment | 5 weeks after the treatment | 12 weeks after the treatment | Reference value |
|---|---|---|---|---|---|---|
| Alanine aminotransferase | 22.1 | 25.9 | 38.7 | 23.3 | 18.1 | 0-60.0 U/L |
| Aspartate aminotransferase | 14.2 | 13.8 | 16 | 16.2 | 15 | 0-40.0 U/L |
| Total bilirubin | 10.22 | 7.98 | 8.52 | 8.68 | 11.14 | 2.00-20.50 µmol/L |
| Alkaline phosphatase | 98.3 | 74.2 | 100.8 | 110.9 | 108.3 | 45-125U/L |
| Albumin | 36.6 | 35.5 | 37.18 | 39.8 | 44.2 | 40.00-55.00 g/L |
| Urea | 4.78 | 3.65 | 4.93 | 5.84 | 4.75 | 1.70-8.30 mmol/L |
| Creatinine | 61 | 65.2 | 48 | 48.8 | 53.7 | 42.0-104.0 µmol/L |
| Blood platelet | 352 | 361 | 406 | 296 | 281 | 100-300 10^{∗}9/L |
| Sodium | 132 | 130.3 | 134.6 | 133 | 135 | 137-147 mmol/L |

The above data prove that the genetically engineered bacteria VNP20009-M of the present invention is used for treating lung cancer, can effectively kill the lung squamous cancer cells and eliminate the tumor lesions, and have no obvious toxic and side effects on the human body.

It is apparent that the above-described embodiments are merely illustrative of the examples and are not intended to limit the embodiments. Other variations or modifications of the various forms may also be made by those of ordinary skill in the art in light of the above description. There is no need and no way to exhaust all of the embodiments. And the obvious variations or modifications derived therefrom are still in the protection scope created by the present invention.

## Claims

1. Application of genetically engineered bacteria VNP20009-M in preparation of drugs for preventing and treating lung cancer.

2. The application according to claim 1, **characterized in that**, the lung cancer includes lung primary tumor, recurrent tumor after lung cancer surgery and lung cancer metastatic tumor.

3. The application according to claim 1 or 2, **characterized in that**, the lung cancer includes non-small cell lung cancer and small cell lung cancer.

4. The application according to claim 3, **characterized in that**, the non-small cell lung cancer includes squamous carcinoma, adenocarcinoma, adenosquamous carcinoma, large cell lung cancer or undifferentiated carcinoma.

5. The application according to any one of claims 1-4, **characterized in that**, the genetically engineered bacteria VNP20009-M have a minimum effective administration dose of 3.5^{∗}10⁷ CFU/M².

6. The application according to any one of claims 1-5, **characterized in that**, administration manners of the genetically engineered bacteria VNP20009-M comprise oral administration, local administration and injection administration.

7. The application according to any one of claims 1-6, **characterized in that**, the genetically engineered bacteria VNP20009-M are attenuated salmonella typhimurium VNP20009 cloned with a L-methioninase gene.

8. The application according to claim 7, **characterized in that**, the genetically engineered bacteria VNP20009-M are attenuated salmonella typhimurium VNP20009 carrying a plasmid, wherein the plasmid is cloned with the L-methioninase gene.

9. The application according to claim 8, **characterized in that**, the plasmid comprises a pSVSPORT plasmid, a pTrc99A plasmid, a pcDNA3.1 plasmid, a pBR322 plasmid or a pET23a plasmid.

10. The application according to any one of claims 7-9, **characterized in that**, the genetically engineered bacteria VNP20009-M are constructed by subcloning the L-methioninase gene into the plasmid to obtain L-methioninase expression plasmid, and electrotransforming the L-methioninase expression plasmid into the attenuated salmonella typhimurium VNP20009.
